# EUROPEAN PATENT APPLICATION

(11) **EP 2 080 507 A1**
(43) Date of publication of application: **22.07.2009**
(21) Application number: 08000602.6
(22) Date of filing: 15.01.2008
(51) Int. Cl.: A61K 9/00, A61K 31/439

(54) **Pharmaceutical formulations comprising an anticholinergic drug**

(71) Applicant: CHIESI FARMACEUTICI S.p.A., I-43100 Parma (IT)
(72) Inventor: Bonelli, Sauro, 43100 Parma (IT); Dagli Alberi, Massimiliano, 43100 Parma (IT)
(74) Representative: Minoja, Fabrizio

(57) **Abstract**

The invention relates to a pharmaceutical formulation suitable to be administered by pressurised metered dose inhalers (pMDIs) comprising a salt of 3-[[[(3-fluorophenyl)[(3,4,5-trifluoro phenyl)methyl]amino] carbonyl]oxy]-1-[2-oxo-2-(2-thienyl)ethyl]-1-azoniabicyclo [2.2.2]octane.

The invention also relates to the process for the preparation thereof and to a pressurised metered dose inhaler filled with said pharmaceutical formulation.

## Description

### FIELD OF THE INVENTION

The invention relates to a pharmaceutical formulation suitable to be administered by pressurised metered dose inhalers (pMDIs ) comprising a salt of 3-[[[(3-fluorophenyl)[(3,4,5-trifluorophenyl)methyl]amino]carbonyl]-oxy]-1-[2-oxo-2-(2-thienyl)ethyl]-1-azoniabicyclo [2.2.2]octane.

The invention also relates to the process for the preparation thereof, and to a pressurised metered dose inhaler filled with said pharmaceutical formulation.

### BACKGROUND TO THE INVENTION

Quaternary ammonium salts acting as muscarinic receptors antagonists are currently used in therapy to induce bronchodilation for the treatment of respiratory diseases and in particular inflammatory or obstructive airway diseases such as asthma and chronic obstructive pulmonary disease (COPD).

For treating chronic diseases, it is often desirable to utilize antimuscarinic drugs with a long-lasting effect. This ensures that the concentration of the active substance necessary for achieving the therapeutic effect is present in the lungs for a long period of time, without the need for the active substance to be administered repeatedly and too frequently.

In particular, it would be highly desirable to utilize antimuscarinic drugs which are therapeutically efficacious upon administration by inhalation once a day.

In order to fulfill such a requirement, antimuscarinic drugs shall exhibit good selectivity for M3 muscarinic receptors, and slow dissociation from them.

Recently it has been reported that tiotropium bromide, the first drug of a new generation of antimuscarinic drugs, exhibits a very slow dissociation from M3 receptors. This behaviour is thought to account for its long lasting activity. However, tiotropium bromide still retains a slow dissociation kinetics for the M2 muscarinic receptors. Since M2 receptors are a major population in the cardiac muscle, a therapy with said drug might be accompanied by undesired cardiac side effects.

The quaternary ammonium salt of 3-[[[(3-fluorophenyl)[(3,4,5-trifluoro phenyl)methyl] amino] carbonyl] oxy]-1-[2-oxo-2-(2-thienyl)ethyl]-1-azoniabicyclo [2.2.2] octane (hereinafter referred to as compound **1**) is a novel compound which has been disclosed in the co-pending Patent Application no. PCT/EP2007/057585. The preparation of the (R) enantiomer of compound **1** is detailed in Example **1** below.

The compound **1** has the following chemical structure: 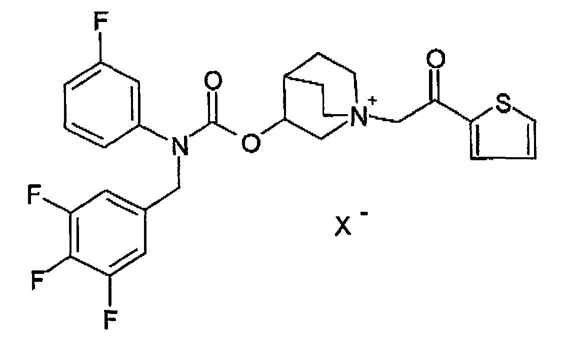 wherein X- is a pharmaceutically acceptable anion, preferably selected from the group consisting of chloride, bromide, iodide, sulfate, phosphate, methanesulfonate, nitrate, maleate, acetate, citrate, fumarate, tartrate, oxalate, succinate, benzoate and p-toluenesulfonate.

In particular the chloride salt of compound **1,** has been found to be equieffective to tiotropium bromide in terms of receptor potency and duration of action, but significantly short-acting on the M2 receptors.

Therefore a salt of compound **1** might provide significant therapeutic benefit in the treatment of respiratory diseases such as asthma and COPD (chronic obstructive pulmonary disease), when administered by inhalation.

Antimuscarinic drugs are currently administered to the respiratory tract by inhalation by means of pressurised metered dose inhalers (pMDIs) which use a hydrofluoroalkane (HFA) propellant to expel the active ingredient as an aerosol, or by means of dry powder inhalers (DPIs).

The aim of the present invention is to provide a HFA based aerosol composition that comprises a pharmaceutically acceptable salt of 3-[[[(3-fluorophenyl)[(3,4,5-trifluorophenyl)methyl]amino]carbonyl]oxy]-1-[2-oxo-2-(2-thienyl)ethyl]-1-azoniabicyclo [2.2.2]octane as active ingredient.

Optimally, said formulation shall be homogeneous, chemically and physically stable and shall be able of providing a therapeutically active dose of the active ingredient.

### SUMMARY OF THE INVENTION

In one aspect the present invention provides a pharmaceutical formulation suitable for aerosol administration by a pressurised metered dose inhaler (pMDI) comprising a pharmaceutically acceptable salt of 3-[[[(3-fluorophenyl)[(3,4,5-trifluorophenyl)methyl]amino]carbonyl]oxy]-1-[2-oxo-2-(2-thienyl)ethyl]-1-azoniabicyclo [2.2.2]octane (compound **1**) and a propellant.

In a particular embodiment said pharmaceutical formulation may be in the form of suspension of particles of micronised crystalline compound **1** in said propellant.

In an alternative embodiment said pharmaceutical formulation may be in the form of solution wherein compound **1** is dissolved in a mixture of said propellant and a suitable amount of a co-solvent such as ethanol.

According to another aspect, the present invention provides a pressurised metered dose inhaler (pMDI) comprising a canister filled with the pharmaceutical formulation of the invention and a metering valve for delivering a full single therapeutically effective dose of the active ingredient.

The invention also relates to the use of one of the formulations described before as a medicament.

In a further aspect, the present invention comprises the use of the formulation described before for the prevention and/or treatment of an inflammatory or obstructive airways disease such as asthma or chronic obstructive pulmonary disease (COPD).

In a still further aspect, the present invention comprises a method of preventing and/or treating an inflammatory or obstructive airways disease such as asthma or chronic obstructive pulmonary disease (COPD), which comprises administration by inhalation of an effective amount of one of the formulations described before.

### DEFINITIONS

The terms 'active drug', 'active ingredient', 'active', 'active compound' 'active substance', and 'therapeutic agent' are used as synonyms.

The terms 'muscarinic receptor antagonists', antimuscarinic drugs' and 'anticholinergic drugs' are used as synonyms.

As used herein, the expressions '% w/w' and '% w/v' mean the weight percentage of the component with respect to the total weight or the total volume of the composition, respectively. The '% w/w' corresponding to the '% w/v' can be calculated by determining the vehicle density.

'Full single therapeutically effective dose' means the quantity of active ingredient administered at one time by inhalation upon actuation of the inhaler.

Said full single dose may be delivered in one, two or more actuations, preferably one or two actuations (shots) of the inhaler.

'Actuation' means the release of the active ingredient from the device by a single activation (e.g. mechanical or breath).

As used herein, the term 'substantially pure' means an active ingredient having an optical purity higher than 95% w/w, preferably higher than 98% w/w.

As used herein, the term 'mass median diameter' means the diameter of 50 percent by weight of the particles.

As used herein, the term 'co-solvent' means a substance having a higher polarity than that of the propellant, and a vapor pressure at 25°C not less than 3 kPa, more preferably not less than 5 kPa.

As used herein, the term 'low volatility component' means a component of the formulation having a vapor pressure at 25°C lower than that of the co-solvent, and preferably not more than 0.1 kPa.

As used herein, the term 'apparent pH' refers to the pH value of a non aqueous medium such as that consisting of a HFA propellant and ethanol. It can be determined according to procedures known to the person skilled in the art.

As used herein, the term 'strong concentrated' refers to an acid which is 100% ionised in an aqueous solution and which is used in a concentration equal to or higher than 1 M.

As used herein, the expression 'formulation chemically stable' means a formulation wherein the stability and the shelf-life of the active ingredient meet the requirements of the ICH Guideline Q1A referring to "Stability Testing of new Active Substances (and Medicinal Products)".

As used herein, in the context of the suspension formulations, the expression 'physically stable' refers to formulations which exhibit substantially no growth in particle size or change in crystal morphology of the active ingredient over a prolonged period, are readily redispersible, and upon redispersion, do not flocculate so quickly as to prevent reproducing dosing of the active ingredient.

As used herein, in the context of the solution formulations, the expression 'physically stable' refers to formulations which exhibit substantially no visually detectable precipitation of the active ingredient over a prolonged period, for example over at least three months, preferably for at least 6 months.

### BRIEF DESCRIPTION OF THE DRAWINGS

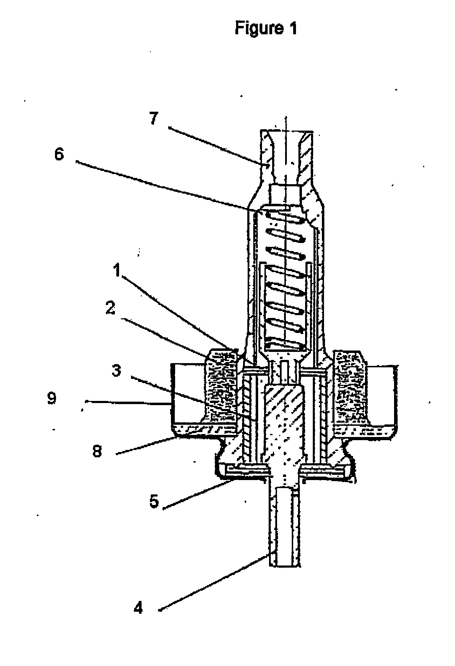

Figure 1 is a cross-sectional view of a typical metering valve.

### DETAILED DESCRIPTION OF THE INVENTION

The compositions of the invention are pharmaceutical formulations suitable for aerosol administration by a pressurised metered dose inhaler (pMDI) comprising a pharmaceutically acceptable salt of 3-[[[(3-fluorophenyl)[(3,4,5-trifluorophenyl)methyl]amino]carbonyl]oxy]-1-[2-oxo-2-(2-thienyl)ethyl]-1-azoniabicyclo[2.2.2]octane (compound **1**) as active ingredient and a propellant.

The compound **1** has the following structure: 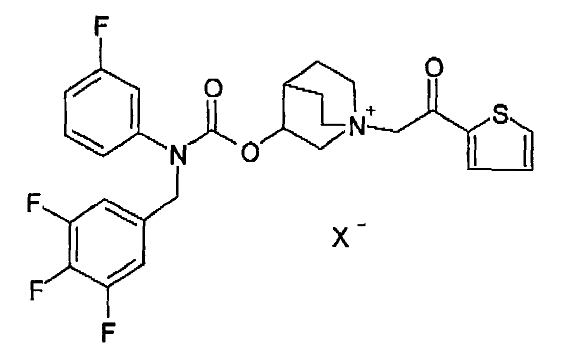 wherein X- is a pharmaceutically acceptable anion, preferably selected from the group consisting of chloride, bromide, iodide, sulfate, phosphate, methanesulfonate, nitrate, maleate, acetate, citrate, fumarate, tartrate, oxalate, succinate, benzoate and p-toluenesulfonate.

Compound **1** is preferably used in the form of chloride salt.

Any pressure-liquefied propellant may be used, preferably a hydrofluoroalkane (HFA). Examples of HFA propellants include 1,1,1,2-tetrafluoroethane (HFA134a) and 1,1,1,2,3,3,3-heptafluoro-n-propane (HFA227) and mixtures thereof.

The preferred propellant is 1, 1, 1, 2-tetrafluoroethane (HFA134a).

The pharmaceutical formulations according to the invention, depending on volume of the metering valve to be used, may suitably comprise from about 0.02 mg to about 2 mg of compound **1** per ml, preferably from 0.1 mg to 0.8 mg per ml.

It will be apparent that compound **1** displays an asymmetric carbon on the quinuclidine ring, and hence may be in the form of a mixture of two optical stereoisomers, (3R)- and (3S)-stereoisomers.

In the preferred embodiments compound **1** is in the form of the substantially pure (3R)-enantiomer.

The (3R)-enantiomer of compound **1** in the form of chloride salt is hereinafter referred to as compound **1'.**

The compositions according to the invention comprises compound **1'** in an amount such that, in case of administration by inhalation from inhalers, the full single therapeutically effective dose (hereinafter simply indicated as the single dose) is advantageously comprised between about 1 µg and about 80 µg, preferably between about 2 µg and about 40 µg.

Said dose will depend on the kind and the severity of the disease and the conditions (weight, sex, age) of the patient and will be administered one or more times a day, preferably once a day.

The single dose may be delivered in one or two or more actuations (shots) of the inhaler wherein the pharmaceutical composition is contained.

For example, a 10 µg single dose may be administered in one shot of 10 µg or as two shots of 5 µg dose.

In one embodiment, the single dose of a pharmaceutical composition comprising compound **1'** is comprised between 2 µg and 10 µg. In other embodiments, the single dose is comprised between 10 µg and 15 µg or between 15 µg and 20 µg.

In further embodiments the single dose is comprised between 20 µg and 40 µg.

In certain embodiments the single dose is 5 µg, in other ones is 10 µg or 20 µg or 40 µg.

The quantities of compound **1** in the compositions which are administered per single dose can be calculated analogously if instead of compound **1',** another salt is used.

In an aspect of the invention the pharmaceutical formulations may be in the form of suspension.

Suspension aerosol formulations refer to formulations in which compound **1** is crystalline, is in particulate form, and is substantially insoluble in the formulation.

The particles of compound **1** present in the formulations shall be in a micronised form so as to permit inhalation of the active ingredient into the lungs upon administration of the aerosol formulation.

Advantageously the particles of the active ingredient shall have a mass median diameter (MMD) of less than 10 micron, preferably in the range of 1 to 10 micron, more preferably between 1 and 6 microns.

In certain embodiments the propellant may consist of HFA 134a, while in other embodiments, the propellant may consist of HFA 227.

The suspension formulation may comprise additional excipients. Excipients suitable for MDI formulations are well known to the skilled person in the art.

In a particular embodiment the suspension formulations may comprise a surfactant. Suitable surfactants are known in the art and include polysorbate 20, polysorbate 80, isopropyl myristate, oleic acid and sorbitan trioleate, and lecithin.

The amount of surfactant, which may be present in the formulation according to the invention, is usually in the range of about 0.001 to 1.0% (w/w), preferably between 0.005 to 0.5% (w/w).

Optionally a wetting agent may be present in the suspension formulations. The wetting agent may assist in the stability and in the manufacturing of the formulation, and is preferably present in a concentration range which does not lead to dissolution of the drug in the propellant formulation.

For example ethanol may be used in a concentration below 1% (w/w), preferably between 0.1 % and 0.5% (w/w).

In other embodiments the formulations according to the invention may additionally comprise further excipients. Examples of excipients which may be mentioned in connection with the invention are sugars such as lactose, amino acids such as alanine, betaine, cysteine, and/or antioxidants such as ascorbic acid, citric acid, sodium edetate, editic acid, tocopherols, butylhydroxytoluene, butylhydroxyanisol and ascorbyl palmitate.

The weight ratio of the drug to the excipient is generally in the range of 1:0.1 to 1:100.

In another aspect the pharmaceutical formulations of the invention may be in the form of solution.

Solution aerosol formulations refer to formulations in which compound **1** is fully dissolved in a mixture of a propellant and a co-solvent.

In said formulations, depending on volume of the metering valve to be used, compound **1** may be present in a concentration comprised between 0.002 and 0.2% (w/w), preferably from 0.01 and 0.08% (w/w). In certain embodiments, the concentration is comprised between 0.003 and 0.054% (w/w).

The co-solvent has a higher polarity than that of the propellant and may include one or more materials.

Advantageously the co-solvent is selected from the group of lower branched or linear alkyl (C₁-C₄) alcohols such as ethanol and isopropyl alcohol, preferably ethanol.

The concentration of the co-solvent will vary depending on the final concentration of the active ingredient in the formulation and on the type of propellant.

For example ethanol may be used in a concentration comprised between 5 and 25% (w/w), preferably between 8 and 22% (w/w), more preferably between 10 and 20% (w/w) and even more preferably 15% (w/w).

The pharmaceutical formulations of the invention may optionally comprise a low volatility component in order to either increase the mass median aerodynamic diameter (MMAD) of the aerosol particles on actuation of the inhaler and/or improve the solubility of the active ingredient in the propellant/co-solvent mixture.

For instance glycols such as propylene glycol, polyethylene glycol and glycerol are particularly suitable for both increasing the MMAD and improving the solubility.

The amount of glycol may vary between 0.1 and 10% w/w, preferably between 0.5 and 5% (w/w), more preferably between 1 and 2% (w/w).

In another embodiment an amount of water comprised between 0.005 and 0.5% (w/w) may be added to the formulations in order to favorably affect the solubility of the active ingredient without increasing the MMAD of the aerosol droplets upon actuation.

The pH of the aerosol solution formulation of the invention may be adjusted to an apparent value comprised between 2.5 and 5.5, preferably between 3.0 and 5.0.

Strong concentrated mineral acids such as hydrochloric acid, nitric acid, sulphuric acid and phosphoric acid are preferably used to adjust the apparent pH.

Phosphoric acid is particularly preferred.

The amount of acid to be added to reach the desired apparent pH will depend on the concentration of the active ingredient and the amount of the co-solvent.

In certain embodiments, the apparent pH may be adjusted by adding phosphoric acid at a concentration comprised between 60 and 85% (from about 10 M to about 15 M) in an amount comprised between 0.0004 and 0.040%, preferably comprised between 0.0008 and 0.020%, more preferably between 0.001 and 0.010% (w/w).

In a particular embodiment the apparent pH is adjusted by adding an amount of 85% phosphoric acid (15.2 M) comprised between 0.0013 and 0.0075% w/w, more preferably comprised between 0.002% and 0.0054% (w/w), even more preferably of 0.0027% (w/w).

The control of pH may favor the chemical stability of the formulations.

It has been found that the stability of the formulation of the invention may also depend on the type of metering valve used with the metered dose inhaler in which the formulation is contained.

The formulation of the invention shall be filled into devices suitable for delivering pressurised pharmaceutical aerosol formulations, hereinafter referred to as pMDI devices.

Said devices comprise a canister fitted with a metering valve. Actuation of the metering valve allows a small portion of the spray product to be released.

Part of all of the internal surfaces of the canister may be made of a metal, for example aluminum or stainless steel or anodized aluminum.

Alternatively the canister may have part of all of the internal surfaces made of anodized aluminum, stainless steel or lined with an inert organic coating. Examples of preferred coatings are epoxy-phenol resins, perfluorinated polymers such as perfluoroalkoxyalkane, perfluoroalkoxyalkylene, perfluoroalkylenes such as poly-tetrafluoroethylene (Teflon), fluorinated-ethylene-propylene, polyether sulfone and a copolymer fluorinated-ethylene-propylene polyether sulfone. Other suitable coatings could be polyamide, polyimide, polyamideimide, polyphenylene sulfide or their combinations.

In certain embodiments canisters having the internal surface lined with Teflon may preferably be used.

In other particular embodiments canisters made of stainless steel may preferably be used.

The canister is closed with a metering valve for delivering a full single therapeutically effective dose of the active ingredient.

A cross-sectional view of a typical metering valve is reported for exemplification purposes in Figure 1.

Generally the metering valve assembly comprises a ferrule **(9)** having an aperture formed therein, a body molding **(7)** attached to the ferrule which houses the metering chamber **(3),** a stem constituted of a core **(1)** and a core extension **(4),** an inner- **(2)** and an outer seal **(5)** around the metering chamber, a spring **(6)** around the core, and a gasket **(8)** to prevent leakage of propellant through the valve.

The gasket may comprise any suitable elastomeric material such as, for example, low density polyethylene, chlorobutyl, black and white butadiene-acrylonitrile rubbers, butyl rubber, neoprene, EPDM (a polymer of ethylenepropylenediene monomer) and TPE (thermoplastic elastomer), preferably EPDM rubbers.

Suitable valves are commercially available from well known manufacturers, for example, from Valois, France, Bespak, plc UK and 3M, Neotechnic Ltd UK.

In general terms the valve seals, especially the gasket seal **(8),** as well as the seals **(2)** and **(5),** shall preferably consists of a material which is inert to and resists extraction into the contents of the formulation, especially when the contents include ethanol.

Advantageously the material of the metering chamber **(3)** is inert to and may resist distortion by contents of the formulation. Particularly suitable materials for use in manufacture of the metering chamber include polyesters e.g. polybutyleneterephthalate (PBT) and acetals, especially PBT.

According to a preferred embodiment, the material of all the internal surface of the canister as well as the material of the metering chamber **(3),** the core **(1),** the core extension **(4),** the spring **(6)** and the body **(7)** of the valve may be substantially or completely made of a metal, preferably of stainless steel.

It has been indeed found that, although the formulations of the invention are chemically stable in devices wherein the metering valves are made of plastic materials such as polyesters or acetals, nevertheless compound **1,** in particular when the device is stored inverted, might tend to adhere onto the surface of said plastic material, giving rise to a loss in the delivered dose, and hence to a potential variability of the therapeutic profile of the drug.

Metering valves made of stainless steel are available under the registered name of Spraymiser™.

The formulation shall be actuated by a metering valve able of delivering a volume of between 50 µl and 100 µl e.g 50 µl, 63 µl. or 100 µl.

Advantageously, the MDI device filled with the formulation may be equipped with a dose counter. For instance, said types of devices are described in EP 1758631 and EP 1787668.

Conventional bulk manufacturing methods and machinery may be employed for the preparation of large scale batches for the commercial production of filled canisters.

For example, the aerosol suspension formulations according to the invention may be prepared by adding the active ingredient to a chilled propellant or optionally a pre-mixed blend of propellant and optionally further excipients and then dispersing the resulting suspension using a suitable mixer. After homogenization the suspension can be filled into the MDI canister which is closed by crimping a metering valve on the canister.

Alternatively the active ingredient and optionally further excipients can be added to a vessel. The liquefied propellant is then introduced into the vessel under pressure and the active ingredient is dispersed and homogenised using a suitable mixer and homogenizer. After homogenization the bulk formulation can be transferred into the individual MDI canisters by using valve to valve transfer methods known to the skilled person.

In the case of the aerosol solution formulations of the invention, the active ingredient is added to a charge vessel and a mixture of ethanol, liquefied propellant, and optionally the low volatility component and the pH-adjusting acid is filled under pressure through the charge vessel into a manufacturing vessel. An aliquot of the formulation is then filled through the metering valve into the canister.

In an alternative process, an aliquot of the liquefied formulation is added to an open canister under conditions which are sufficiently cold that the formulation does not vaporize, and then a metering valve crimped onto the canister.

In an alternative process, an aliquot of the active ingredient dissolved in co-solvent, optionally in the presence of the low volatility component and the pH-adjusting acid is dispensed into an empty canister, a metering valve is crimped on, and then the propellant is filled into the canister through the valve.

Preferably, the processes are carried out an in inert atmosphere, for instance by insufflating nitrogen, in order to avoid the uptake of humidity from the air.

Each filled canister is conveniently fitted into a suitable channeling device prior to use to form a metered dose inhaler for administration of the medicament into the lungs of a patient. Suitable channeling devices comprise, for example a valve actuator and a cylindrical or cone-like passage through which medicament may be delivered from the filled canister via the metering valve to the mouth of a patient e.g. a mouthpiece actuator.

In a typical arrangement the valve stem is seated in a nozzle block which has an orifice leading to an expansion chamber. The expansion chamber has an exit orifice which extends into the mouthpiece. Actuator (exit) orifice having a diameter in the range 0.15 - 0.45 mm and a length from 0.30 to 1.7 mm are generally suitable.

Preferably an orifice having a diameter from 0.2 to 0.44 mm may be used, e.g. 0.22 0.25, 0.30, 0.33 or 0.42 mm.

In certain embodiments of the invention, it may be useful to utilize laser-drilled actuator orifices having a diameter ranging from 0.10 to 0.22 mm, in particular from 0.12 to 0.18 mm as those described in WO 03/053501.

The use of said fine orifices may also increase the duration of the cloud generation and hence, may facilitate the coordination of the cloud generation with the slow inspiration of the patient.

In case the ingress of water into the formulation is to be avoided, it may be desired to overwrap the MDI product in a flexible package capable of resisting water ingress.

It may also be desired to incorporate a material within the packaging which is able to adsorb any propellant and co-solvent which may leak from the canister (e.g. a molecular sieve).

Optionally the MDI device filled with the formulation of the invention may be utilised together with suitable auxiliary devices favoring the correct use of the inhaler.

Said auxiliary devices are commercially available and, depending on their shape and size, are known as spacers", "reservoirs" or "expansion chambers".

Volumatic™ is, for instance, one of the most known and used reservoir, while Aerochamber™ is one of the most used and known spacer.

A suitable expansion chamber is reported for example in WO 01/49350.

The formulation of the invention may also be used with common pressurised breath-actived inhalers such as those known with the registered names of Easi-Breathe™ and Autohaler™.

The formulations of the invention may further comprise other therapeutic agents currently used in the treatment of respiratory disorders, e.g. corticosteroids such as budesonide and its epimers, beclometasone dipropionate, triamcinolone acetonide, fluticasone propionate, flunisolide, mometasone furoate, rofleponide and ciclesonide, anticholinergic or antimuscarinic agents such as ipratropium bromide, oxytropium bromide, tiotropium bromide, glycopyrrolate bromide, and the group of phosphodiesterase-4 (PDE-4) inhibitors such as roflumilast and their combinations.

Administration of the formulations of the invention may be indicated for the prevention and/or treatment of mild, moderate or severe acute or chronic symptoms or for prophylactic treatment of respiratory diseases such as asthma and chronic obstructive pulmonary disease (COPD). Other respiratory disorders characterised by obstruction of the peripheral airways as a result of inflammation and presence of mucus such as chronic obstructive bronchiolitis and chronic bronchitis may also benefit by this kind of formulation.

The invention is better illustrated by the following examples.

### Example 1 - Preparation of (3R)-3-[(3-Fluorophenyl)-(3,4,5-trifluoro-benzyl)carbamoyloxy]-1-(2-oxo-2-thiophen-2-yl-ethyl)-1-azoniabicyclo-[2.2.2]octane chloride (compound 1')

A solution of (3R)-(3-fluorophenyl)-(3,4,5-trifluorobenzyl)carbamic acid 1-aza-bicyclo[2.2.2]oct-3-yl ester hydrochloride (prepared as described in WO03/053966 as Intermediate 15) (1 g, 2.248 mmol) in water (5 ml) was added with an excess of sodium carbonate (1 g) in water (4 ml) and extracted with ethyl acetate (2x10 ml). The organic layer was dried over anhydrous sodium sulfate and evaporated under vacuum.

The resulting free base (750 mg, 1.836 mmol) was dissolved in acetonitrile (4 ml) and added dropwise with a solution of 2-chloro-1-thiophen-2-yl-ethanone (750 mg, 3.657 mmol) in chloroform (6 ml). The resulting solution was refluxed for 12 hours. The solvent was evaporated and the residue dissolved in acetonitrile (7.5 ml). Crystallisation occurred after some minutes of stirring. The solid was filtered, washed with acetonitrile (1 ml) and dried under vacuum at 45° C. 760 mg of the title compound was obtained as white solid.

Mother liquors were evaporated to dryness and purified by column chromatography (SiO₂, eluent: dichloromethane/methanol =95/5 v/v to 80/20 v/v) to give further 120 mg of the title compound.

### Example 2

To prepare the suspension formulations according to the invention the crystalline (3R)- 3-[[[(3-fluorophenyl)[(3,4,5-trifluoro phenyl)methyl]amino] carbonyl]oxy]-1-[2-oxo-2-(2-thienyl)ethyl]-1-azoniabicyclo [2.2.2]octane chloride (compound **1'**), obtained as reported in Example 1, is micronised by known methods, to prepare the active substance in the form of particles having a typical particle size suitable for inhalation.

Examples of formulations are reported in Table 1.

**Table 1**

| *Formulation* | *Amounts* | | |
|---|---|---|---|
| | Per unit | | Nominal dose |
| **(a)** | mg | % (w/v) | µg |
| Compound **1'** | 7.62 | 0.064 | 40 |
| Polysorbate 20 | 24 | 0.2 | |
| HFA 134a q.s to 12 ml | - | | |

| **(b)** | | | |
|---|---|---|---|
| Compound **1'** | 3.81 | 0.032 | 20 |
| Polysorbate 20 | 24 | 0.2 | |
| HFA 227 q.s. to 12 ml | - | | |

| **(c)** | | | |
|---|---|---|---|
| Compound **1'** | 1.91 | 0.012 | |
| Oleic acid | 0.6 | 0.005 | |
| HFA 134a q.s. to 12 ml | - | | - |

### Example 3

Aerosol solution formulations are prepared by dissolving compound **1'** in the propellant in the presence of ethanol and optionally phosphoric acid and water according to the methods reported in the description.

Examples of formulations are reported in Table 2.

**Table 2**

| *Formulation* | *Amounts* | | |
|---|---|---|---|
| | Per unit | | Nominal dose |
| **(d)** | mg | % (w/w) | µg |
| Compound **1'** | 0.77 | 0.008 | 5 |
| Ethanol | 1650.0 | 15 | |
| HFA 134a | q.s. to 11,000 | - | |

| **(e)** | | | |
|---|---|---|---|
| Compound **1'** | 1.54 | 0.016 | 10 |
| Ethanol | 1650.0 | 15 | |
| HFA 134a | q.s. to 11,000 | - | |

| **(f)** | | | |
|---|---|---|---|
| Compound **1'** | 1.54 | 0.016 | 10 |
| Ethanol | 1100 | 10 | |
| Water | 55 | 0.5 | |
| HFA 134a | q.s. to 11,000 | - | |

| **(f)** | | | |
|---|---|---|---|
| CHF 5407.01 | 0.30 | 0.003 | 2 |
| Ethanol | 1650.0 | 15 | - |
| Phosphoric acid 15.2 M | 0.3 | 0.0027 | - |
| HFA 134a | q.s. to 11,000 | - | |

| **(g)** | | | |
|---|---|---|---|
| Compound **1'** | 1.50 | 0.014 | 10 |
| Ethanol | 1650.0 | 15 | |
| Phosphoric acid 15.2 M | 0.3 | 0.0027 | |
| HFA 134a | q.s. to 11,000 | - | |

| **(h)** | | | |
|---|---|---|---|
| Compound **1'** | 6.0 | 0.054 | 40 |
| Ethanol | 1650.0 | 15 | |
| Phosphoric acid 15.2 M | 0.3 | 0.0027 | |
| HFA 134a | q.s. to 11,000 | - | - |

### Example 4

The formulations **(f), (g)** and **(h)** of Example 3 are filled in Teflon coated standard aluminum canisters under pressure and fitted with a metering valve having a 63 µl metering chamber. An actuator with an orifice diameter of 0.22 mm is used.

The aerodynamic particle size distribution of each tested formulation is characterised using a Multistage Cascade Impactor according to the procedure described in European Pharmacopoeia 2nd edition, 1995, part V.5.9.1, pages 15-17.

In this specific case, an Andersen Cascade Impactor (ACI) is utilised.

The following parameters are determined:
i) delivered dose which is calculated from the cumulative deposition in the ACl;
ii) respirable dose (fine particle dose) which is obtained from the deposition on Stages 3 (S3) to filter (AF) corresponding to particles ≤ 4.7 microns, divided by the number of actuation per experiment;
iii) respirable fraction (fine particle fraction) which is the ratio between the respirable dose and the delivered dose.

Deposition of the drug on each ACI plate is determined by high pressure liquid chromatography (HPLC).

The delivery characteristics of said formulations are reported in Table 3.

**Table 3**

| | Nominal dose (µg) | Delivered dose (µg) | Respirable dose (µg) | Respirable fraction (%) |
|---|---|---|---|---|
| Formulation **(f)** | 2 | 1.6 | 0.86 | 60.4 |
| Formulation **(g)** | 10 | 8.6 | 4.2 | 55.1 |
| Formulation **(h)** | 40 | 35.5 | 17.5 | 54.5 |

### Example 5 - Assessment of the bronchodilation activity of compound 1'

Airway reactivity is measured using barometric plethysmography (Buxco, USA). Male guinea pigs (500-600 g) are individually placed in plexiglass chambers. After an acclimatisation period, animals are exposed to nebulised saline for 1 min to obtain airway baseline reading. This is followed by a 1 min challenge with nebulised acetylcholine (Ach) -2.5 mg/mL.

After 60 min, 5 min nebulisation of vehicle or the compound **1'** in the range 2.5 -250 mM are applied and Ach challenge is then repeated after 2, 5, 24, 48 and 72 h. Recording of pressure fluctuations in the chambers are taken for 5 min after each nebulisation and analysed to calculate Enhanced Pause (Penh). Airway reactivity is expressed as percentage increase in Penh compared with Penh values from the nebulisation of vehicle.

Two hours after the end of nebulisation with compound **1',** the Ach-induced increase in Penh is dose-dependently inhibited by the compound, with a maximal effect of 99.2 ± 0.5 at 250 mM.

As for the time-course of the effect, compound **1'** shows increasing duration of action with increasing dose.

After inhalation of 250 mM of compound **1',** effect persists unchanged up to 48 h (83.0 ± 16.1%), while at 72 h a residual activity of 34.8 ± 20.9% is present. Twenty-four hours after 25 and 50 mM compound **1'** inhalation, a significant bronchoprotective effect was observed (87.1 ± 8.7% and 63.1 ± 19.2%, respectively). At 50 mM, a significant inhibition persists up to 48 h (49.2 ± 23.2%). Inhalation of lower concentrations results in an effect that did not exceed the 5 h observation point.

The estimation of lung levels of compound **1'** achieved after nebulisation endowed with a submaximal bronchodilator activity at 2 h after treatment reveals that the its retained dose in the target organ is about 50 µg/kg. If an extrapolation of these results from rat to human is made, it can be predicted that in patients the therapeutically effective dose might be comprised between 1 and 80 µg, preferably between 5 and 40 µg, after administration by inhalation.

## Claims

1. A pharmaceutical formulation for aerosol administration by a pressurised metered dose inhaler (pMDI) comprising a pharmaceutically acceptable salt of 3-[[[(3-fluorophenyl)[(3,4,5-trifluoro phenyl)methyl]amino] carbonyl]oxy]-1-[2-oxo-2-(2-thienyl)ethyl]-1-azoniabicyclo [2.2.2]octane (compound **1**).

2. The formulation according to claim 1 wherein the amount of compound **1** is comprised between 0.01 mg and 2 mg per ml.

3. The formulation according to claim 2 wherein the amount is comprised between 0.02 mg and 0.8 mg per ml.

4. The formulation according to claims 1 to 3 further comprising a propellant.

5. The formulation according to claim 4 wherein the propellant is a hydrofluoroalkane selected from the group consisting of 1, 1, 1, 2-tetrafluoroethane (HFA134a) and 1, 1, 1, 2, 3, 3, 3-heptafluoro-n-propane (HFA227) and mixtures thereof.

6. The formulation according to any one of claims 1 to 5 wherein the compound **1** is in the form of chloride salt of the (3R)-enantiomer (compound **1').**

7. The formulation according to claim 6 wherein the active ingredient is administered at a full single therapeutically effective dose comprised between about 1 µg and about 80 µg.

8. The formulation according to claim 7 wherein the single dose is comprised between about 2 µg and about 40 µg.

9. The formulation according to any one of claims 1 to 8 wherein compound **1** is in form of micronised crystalline particles suspended in the propellant.

10. The formulation according to claim 9, further comprising an excipient selected from the group consisting of surfactants, wetting agents or adjuvants selected form the class consisting of sugars, amino acids and antioxidants.

11. The formulation according to claim 10 wherein the surfactant is selected from the group consisting of polysorbate 20 polysorbate 80, oleic acid, sorbitan trioleate and lecithin.

12. The formulation according to any one of claims 1 to 8 further comprising a co-solvent.

13. The formulation according to claim 12 wherein compound **1** is dissolved in the mixture of propellant and said co-solvent.

14. The formulation according to claim 13 wherein the concentration of compound **1** is comprised between 0.01% and 0.08% (w/v).

15. The formulation according to claim 14 wherein the concentration is comprised between 0.003% and 0.054% (w/v).

16. The formulation according to any one of claims 12 to 15, wherein the co-solvent is selected from the group of lower linear or branched alkyl (C₁-C₄) alcohols.

17. The formulation according to claim 16 wherein the co-solvent is ethanol.

18. The formulation according to claim 17 wherein ethanol is present in a concentration comprised between 8% and 22% (w/w).

19. The formulation according to claim 18 wherein the concentration is comprised between 10 and 20% (w/w).

20. The formulation according to claim 19 wherein the concentration is 15% (w/w).

21. The formulation according to any one of claims 13 to 20 wherein the pH of the solution has been adjusted to an apparent value comprised between 2.5 and 5.5.

22. The formulation according to claim 21 wherein the pH has been adjusted by addition of a mineral acid selected from the group consisting of hydrochloric acid, nitric acid, sulphuric acid and phosphoric acid.

23. A pressurised metered dose inhaler comprising a canister filled with the pharmaceutical formulation according to any preceding claim, and a metering valve for delivering a full single therapeutically effective dose of the formulation.

24. The inhaler according to claim 23 wherein part or all of the internal surfaces of the canister are made of a metal selected from the group consisting of aluminium, stainless steel and anodised aluminium.

25. The inhaler according to claim 23 wherein part or all of internal metallic surfaces of the canister lined with an inert organic coating.

26. The inhaler according to claim 25 wherein the inert organic coating is selected from the group consisting of epoxy-phenol resins, perfluoroalkoxyalkane, perfluoroalkoxyalkylene, perfluoroalkylenes such as polytetrafluoroethylene, fluorinated-ethylene-propylene, polyether sulfone and a copolymer fluorinated-ethylene-propylene polyether sulfone.

27. The inhaler according to claim 23 or 24 wherein the metering chamber **(3)** the core **(1),** the core extension **(4),** the spring **(6)** and the body **(7)** of the valve are completely or substantially made of a metal.

28. The inhaler according to claim 27 wherein the metal is stainless steel.

29. The use of a formulation according to any one of claims 1 to 22 as a medicament.

30. The use of a formulation according to any one of claims 1 to 22 for the prevention and/or treatment of an obstructive airways disease.

31. The use according to claim 30 wherein the disease is asthma

32. The use according to claim 30 wherein the disease is chronic obstructive pulmonary disease (COPD).
